# EUROPEAN PATENT APPLICATION

(11) **EP 3 649 871 A1**
(43) Date of publication of application: **13.05.2020**
(21) Application number: 18804503.3
(22) Date of filing: 27.06.2018
(51) Int. Cl.: A23L 2/395, A23L 2/40, A23L 2/52, A23L 33/105

(54) **LYCIUM RUTHENICUM**

(30) Priority: 04.07.2017 CN 201710539532
(71) Applicant: Qinghai Ruiyuan Pharmaceutical Research Institute Co., Ltd., Xining, Qinghai 810003 (CN)
(72) Inventor: GAO, Lin, Xining Qinghai 810003 (CN)
(74) Representative: Cabinet Chaillot
(86) International application number: PCT/CN2018/093035
(87) International publication number: WO 2019/007237

(57) **Abstract**

The invention belongs to the technical field of food processing, and relates to a black Chinese wolfberry effervescent tablet and a preparation method thereof. The black Chinese wolfberry effervescent tablet of the invention is prepared from lyophilized powder of black Chinese wolfberry extract, citric acid, an alkali source, a water-soluble starch, sucrose and an encapsulant as raw materials. The preparation method thereof comprises the following steps: (1) lyophilized powder of black Chinese wolfberry extract and part of citric acid are crushed and mixed; (2) the crushed and mixed material is taken out, added with a water-soluble starch and mixed again, and the resulting mixture is prepared into a soft material by taking ethanol as a wetting agent, then prepared into granules and dried until the water content is controlled at 2.2-3.5%; and (3) the granules prepared in step (2) are mixed with an alkali source, the remaining citric acid, sucrose and an encapsulant and then pressed into effervescent tablets. The effervescent tablet prepared by the invention takes effect rapidly after dissolution, has a long anti-oxidation shelf-life, disintegrates rapidly in water, has a stable color in pink and good taste, and is especially suitable for children, old people, and patients who cannot swallow.

## Description

### FIELD OF THE INVENTION

The invention belongs to the technical field of food processing, and more particularly relates to a black Chinese wolfberry effervescent tablet and a preparation method thereof.

### BACKGROUND OF THE INVENTION

Black Chinese wolfberry, recognized as "nourishing soft gold", is a specific wild plant resource in the western region of China and a plant belonging to the family Solanaceae, which is recorded in the "Records of Uyghur Medicine", "Medical Canon in Four Sections" and "Jingzhu Herbal of Tibetan Medicine". The black Chinese wolfberry contains polysaccharides, amino acids, betaine, carotene, vitamin E, vitamin C, nicotinic acid and other vitamins as well as trace elements such as manganese, chromium, zinc, copper, magnesium, calcium, strontium and cobalt. Particularly, the black Chinese Wolfberry is rich in anthocyanin, with the anthocyanin content being several times or even more than ten times than that of blueberry, is a natural wild plant with the highest anthocyanin content found so far and called as "the king of anthocyanin". Anthocyanin is a natural water-soluble pigment, belongs to flavonoids, has the functions of prevention of cardiovascular and cerebrovascular diseases, anti-cancer, anti-oxidation, anti-fungi, anti-mutation, anti-aging and the like, and can be used as a natural pigment, a natural antioxidant, a nutritional supplement, etc. In modern medicine, it is believed that black Chinese wolfberry has obvious functions of enhanced human immunity, anti-tumor, anti-aging, lowered blood fat, lowered blood sugar, etc. For children with physical weakness, enuresis, growth retardation and poor resistance to colds, the administration of black Chinese wolfberry can significantly improve the immune function, enhance the disease resistance and quickly recover the physical fitness, and can provide significant effects on elimination of eye diseases and stress and restoration of the eye vision. Abundant anthocyanin contained in black Chinese wolfberry is a natural sunlight shield which can effectively prevent the damage of ultraviolet rays to the skin, and proanthocyanidin in black Chinese wolfberry can well resist the external oxidation on cells, and effectively eliminate the accumulation and precipitation of free radicals of harmful substances to the skin, thus delaying the aging of human cells, protecting collagen, reducing wrinkles, making the skin fresh and elastic, and maintaining a young state.

At present, black Chinese wolfberry has attracted more and more attention as a valuable product with both food and medicine functions, and the categories of black Chinese wolfberry products emerging in the market are also increasing year by year. There are many black Chinese wolfberry products with various characteristics made from natural black Chinese wolfberry. CN103431327A discloses a black fruit wolfberry troch and CN104273633A discloses a black Chinese wolfberry effervescent tablet and a preparation method thereof, wherein the black Chinese wolfberry effervescent tablet obtained has the unique flavor of black Chinese wolfberry without bitter taste, and has a mellow and original taste. An effervescent tablet is a novel solid formulation containing an effervescent disintegrant. Two substances in the effervescent disintegrant cannot react without being ionized, but may experience an acid-base reaction when the effervescent tablet is put into water such that the tablet is quickly disintegrated due to a large amount of carbon dioxide bubbles produced. The bubbles produced by disintegration also cause the tablet to roll up and down in water, which accelerates the disintegration of the tablet. Carbon dioxide produced upon the disintegration of the tablet is partially dissolved in the drinking water, allowing the drinking water to have a soda-like taste. The effervescent tablet has the advantages of rapid disintegration, convenience in administration, rapid effect, high bioavailability, improved clinical efficacy, etc. and is especially suitable for children, old people, and patients who have difficulty in swallowing pills. A black Chinese wolfberry effervescent tablet can be quickly disintegrated in cold water and easy to carry, and therefore not only can meet the demand of people on instant foodstuff due to the fast pace of life in modern society, but also can supplement essential biologically active ingredients and trace elements for the human body. The black Chinese wolfberry effervescent tablet has an extremely high nutritional value and a broad market prospect. However, the currently processed black Chinese wolfberry effervescent tablets still have many problems such as unscientific processing methods, low bioavailability, loss of nutrients, poor absorption by the human body and poor taste, especially that the black Chinese wolfberry effervescent tablets in the prior art generally have the problems of unstable color and unclarified solution after effervescence. In order to solve the problem in stability, CN104783292A discloses a black Chinese wolfberry effervescent tablet containing black Chinese wolfberry extract powder added with 5-12% of calcium carbonate by weight of the black Chinese wolfberry powder, which increases the stability of active ingredients in black Chinese wolfberry, but weakens the unique flavor as well as the mellow and original taste of the black Chinese wolfberry effervescent tablet due to the addition of a larger amount of calcium carbonate, thus resulting in poor taste; and moreover, a larger amount of NaCl needs to be added to a PEG coating, which also leads to poor taste.

### SUMMARY OF THE INVENTION

To overcome the above technical problems existing in the prior art, an object of the invention is to provide a black Chinese wolfberry effervescent tablet product which is natural, efficient, convenient, safe, bright in color and good in taste by full utilization of all-natural black Chinese wolfberry resources, and a preparation method thereof.

To achieve the object of the invention, a first aspect of the invention employs the following technical solutions.

A preparation method of a black Chinese wolfberry effervescent tablet prepared from lyophilized powder of black Chinese wolfberry extract, citric acid, an alkali source, a water-soluble starch, sucrose and an encapsulant as raw materials comprises the following steps:
(1) lyophilized powder of black Chinese wolfberry extract and part of citric acid are crushed and mixed;
(2) the crushed and mixed material is taken out, added with a water-soluble starch and mixed again, and the resulting mixture is prepared into a soft material by taking ethanol as a wetting agent, then prepared into granules and dried until the water content is controlled at 2.2-3.5%; and
(3) the granules prepared in step (2) are mixed with an alkali source, the remaining citric acid, sucrose and an encapsulant and then pressed into effervescent tablets.

The raw materials consist of 25-45% of citric acid, 5-10% of an alkali source, 3-7% of a water-soluble starch, 1-3% of an encapsulant, 0.5-5% of sucrose and 40-60% of lyophilized powder of black Chinese wolfberry extract based on the weight percentage.

Preferably, the raw materials consist of 25-35% of citric acid, 8-10% of an alkali source, 3-7% of a water-soluble starch, 1-3% of an encapsulant, 0.5-5% of sucrose and 51-60% of lyophilized powder of black Chinese wolfberry extract.

The lyophilized powder of black Chinese wolfberry extract is prepared as follows: dried fruits of black Chinese wolfberry as a raw material are extracted with an aqueous citric acid solution having a pH value of about 3.5 and filtered to collect a filtrate, and the filtrate is concentrated followed by lyophilization.

The lyophilized powder of black Chinese wolfberry extract is prepared as follows: dried fruits of black Chinese wolfberry as a raw material are added to an aqueous citric acid solution having a weight 3-5 times that of the dried fruits of black Chinese wolfberry and a pH of about 3.5 while stirring, soaked at normal temperature for half an hour and then heated up to about 90°C for extraction, then a filtrate is collected through filtration, a filter residue is then added to an aqueous citric acid solution having a weight 3-5 times that of the filter residue and a pH of about 3.5, the raw material is continuously stirred and heated up to about 90°C for extraction, then a filtrate is collected through filtration, and the two filtrates collected are combined and concentrated followed by lyophilization and superfine crushing to above 300 meshes.

The alkali source is prepared as follows: sodium carbonate and/or sodium bicarbonate are/is melted with polyethylene glycol (e.g. PEG 6000), then encapsulated and fed into an oven, dried at below 40°C and then crushed, wherein the amount of the polyethylene glycol used is 5-10 wt% of the sodium carbonate and/or sodium bicarbonate.

The encapsulant is preferably superfinely crushed polyethylene glycol (e.g. commonly used PEG-6000)

The superfinely crushed polyethylene glycol is prepared by superfine crushing of sheet-like polyethylene glycol at a temperature of below 40°C for more than 5 min using a superfine crushing vibration mill.

A second aspect of the invention also relates to a black Chinese wolfberry effervescent tablet prepared by the above preparation method.

Compared with the prior art, the black Chinese wolfberry effervescent tablet obtained by the preparation method of the invention has the following beneficial effects:
the effervescent tablet prepared from superfine lyophilized powder of black Chinese wolfberry extract as a main raw material dissolves rapidly and fully, enables the human body to fully absorb its active ingredients, can have a shorter brewing time than traditional effervescent tablets, is convenient to store and carry, takes effect rapidly after dissolution, has a long anti-oxidation shelf-life, undergoes a rapid acid-base disintegration reaction in water, has a stable color (pink) and
good taste, and is especially suitable for children, old people, and patients who cannot swallow; and
the effervescent tablet is natural, efficient, convenient, safe, good in taste and the like.

### DETAILED DESCRIPTION OF THE INVENTION

The black Chinese wolfberry effervescent tablet and the preparation method thereof according to the invention will be further described below in conjunction with particular examples so as to help those skilled in the art to have a more complete, accurate and in-depth understanding of the inventive concepts and technical solutions of the invention.

The black Chinese wolfberry effervescent tablet of the invention is prepared by adding citric acid, an alkali source, a water-soluble starch, optional sucrose (e.g. sucralose) and an optional encapsulant (e.g. superfinely crushed PEG6000) to lyophilized powder of black Chinese wolfberry extract as a main raw material. The preparation process is as follows: firstly, lyophilized powder of black Chinese wolfberry extract and part of citric acid are placed in a superfine crushing vibration mill and superfinely mixed for 5 min at a temperature controlled at below 40°C, the fully mixed material is taken out, added with a water-soluble starch and mixed again, and the resulting mixture is prepared into a soft material by taking 85% (V/V) ethanol (added in an amount of 30 wt% of the powder amount) as a wetting agent; granules are prepared by a 15-mesh standard sieve and dried in a hot air circulating oven at 40°C until the water content is about 3.0%; and finally, the granules are mixed with an alkali source, the remaining citric acid, sucrose and an encapsulant, fed into a tablet press and pressed into effervescent tablets at an ambient humidity of ≤30% by the tablet press. The raw materials consist of 25-45% of citric acid, 5-10% of an alkali source, 3-7% of a water-soluble starch, 1-3% of an encapsulant, 0.5-5% of sucrose and 40-60% of lyophilized powder of black Chinese wolfberry extract based on the weight percentage.

The lyophilized powder of black Chinese wolfberry extract is prepared as follows: dried fruits of black Chinese wolfberry as a raw material are firstly added to an aqueous citric acid solution having a weight 3-5 times that of the dried fruits and a pH of 3-4 (preferably 3.5), then continuously stirred for half an hour and then heated up to microboiling (about 90°C) for 1 h extraction, then a filtrate is collected through filtration, a filter residue is then added to an aqueous citric acid solution having a weight 3-5 times that of the filter residue and a pH of 3-4 (preferably 3.5), the raw material is continuously stirred and heated up to 90°C for 40 min extraction, then a filtrate is collected through filtration, and the two filtrates collected are combined and concentrated followed by lyophilization and superfine crushing to above 300 meshes.

The alkali source is preferably selected from sodium carbonate and/or sodium bicarbonate which are/is melted with 5% PEG 6000, then encapsulated and fed into an oven, dried at 40°C and then crushed to pass through a 80-mesh sieve.

The PEG-6000 is prepared by superfine crushing of sheet-like PEG-6000 at a temperature of below 40°C for 5 min using a superfine crushing vibration mill.

Compared with the prior art (for example, the prior art mentioned in the background section), the invention has the following significant advantages:
(1) the dried fruits of black Chinese wolfberry used as a raw material in the invention are natural with abundant resources, and need to be vigorously developed and utilized to increase the utilization value of the raw material used;
(2) the processes such as stirring and leaching, lyophilization, superfine crushing and superfine mixing employed in the invention are processing technologies in the fields of modern food and medicine, and the process steps and process methods employed are rational, scientific and easy to operate;
(3) the effervescent tablet of the invention prepared from superfine lyophilized powder of black Chinese wolfberry as a main raw material dissolves rapidly and fully, enables the human body to fully absorb its active ingredients, can have a shorter brewing time than traditional effervescent tablets, is convenient to store and carry, is convenient and quick to drink, takes effect rapidly after dissolution, and has a long anti-oxidation shelf-life;
(4) the product of the invention can ensure the maximized effect of the active ingredients of the product, has high bioavailability, has no discards left, is suitable for industrial production, and can greatly increase the added value of agricultural products, thereby promoting the planting area of agricultural products, forming large-scale planting and improving the output value effect of agricultural and sideline products; and
(5) the effervescent tablet of the invention has a suitable dosage form, disintegrates rapidly in an acid-base reaction in water, has a clear solution and a stable color (bright purple), facilitates improving the bioavailability of drugs, has good taste, and is suitable for all kinds of people, especially children, old people, and patients who cannot swallow.

The black Chinese wolfberry effervescent tablet obtained by the preparation method of the invention has the following characteristics:
"beautiful"-after the black Chinese wolfberry effervescent tablet disintegrates in water, its color is pink naturally formed by anthocyanin of black Chinese wolfberry fruits in acidic water, which looks very beautiful and easily attracts the drinking desire of people;
"interesting"-upon disintegration in water, the black Chinese wolfberry effervescent tablet is pinkish, and the color constantly changes, thus continuously releasing colored bubbles and finally forming pink; and
"delicious"-the brewed black Chinese wolfberry effervescent tablet water is slightly sweet and not bitter with good taste, wherein the black Chinese wolfberry effervescent tablet is economical, and nearly a hundred people who have drunk its test samples agree that both the taste and color are preferable, and therefore the effervescent tablet is suitable for both young and old people.

### Example 1

Dried fruits of black Chinese wolfberry, citric acid, sodium carbonate, PEG6000, a water-soluble starch and sucralose were taken as raw materials for use.

A proper amount of harvested dried fruits of black Chinese wolfberry which had been washed clean and freed of impurities were weighed and added to an aqueous citric acid solution having a weight 3-5 times that of the dried fruits and a pH of about 3.5; the raw material was continuously stirred until the aqueous solution changed color, then cold-soaked for half an hour and then heated up to microboiling (about 90°C) for 1 h extraction; then a filtrate was collected through filtration; a filter residue was then added to an aqueous citric acid solution having a weight 3-5 times that of the filter residue and a pH of about 3.5; the raw material was continuously stirred and heated up to 90°C for 40 min extraction; then a filtrate was collected through filtration; and the two filtrates collected were combined and concentrated followed by lyophilization and then superfine crushing to above 300 meshes to prepare lyophilized powder of black Chinese wolfberry extract for use.

Sodium carbonate and PEG-6000 were weighed; and sodium carbonate was melted with PEG6000 (the amount of PEG6000 used was 5 wt% of sodium carbonate), then encapsulated and fed into an oven, dried at 40°C and then crushed to prepare a 80-mesh alkali source for use.

The raw material PEG-6000 was additionally weighed, placed in a superfine crushing vibration mill, superfinely crushed at below 40°C for 5 min and then taken out for use. 51 kg of the lyophilized powder of black Chinese wolfberry extract prepared above was weighed, firstly added together with 10 kg of citric acid to the superfine crushing vibration mill and superfinely mixed for 5 min at a temperature kept at 40°C; the superfinely mixed material was taken out, placed in a trough-type mixer, added with 6 kg of a water-soluble starch and mixed for 30 min; the resulting mixture was prepared into a soft material by adding 85% (V/V) ethanol (in an amount of 30% of the powder amount) as a wetting agent; a 15-mesh standard sieve was mounted on an oscillating granulator to prepare the soft material into granules; the granules was fed into a hot air circulating oven based on 1 kg/tray and dried at 40°C until the water content was about 3.0%; and the oven-dried granules was taken out and placed in a 15-mesh oscillating granulator for granulation.

Subsequently, the resulting granules were mixed with 8 kg of the crushed alkali source, 23 kg of the remaining citric acid, 1 kg of sucralose and 1 kg of the superfinely crushed PEG-6000 in a three-dimensional mixer for 1 h; and the fully mixed granules were taken out, fed into a tablet press, and prepared into the black Chinese wolfberry effervescent tablet of this Example at an ambient humidity of ≤30%, a temperature of 20°C, a tablet press pressure of 9 kN and a rotational speed of 10 r/min.

### Example 2

Dried fruits of black Chinese wolfberry, citric acid, sodium carbonate, PEG6000, a water-soluble starch and sucralose were taken as raw materials for use.

A proper amount of harvested dried fruits of black Chinese wolfberry which had been washed clean and freed of impurities were weighed and added to an aqueous citric acid solution having a weight 3-5 times that of the dried fruits and a pH of about 3.5; the raw material was continuously stirred until the aqueous solution changed color, then cold-soaked for half an hour and then heated up to microboiling (about 90°C) for 1 h extraction; then a filtrate was collected through filtration; a filter residue was then added to an aqueous citric acid solution having a weight 3-5 times that of the filter residue and a pH of about 3.5; the raw material was continuously stirred and heated up to 90°C for 40 min extraction; then a filtrate was collected through filtration; and the two filtrates collected were combined and concentrated followed by lyophilization and then superfine crushing to above 300 meshes to prepare lyophilized powder of black Chinese wolfberry extract for use.

Sodium carbonate and PEG-6000 were weighed; and sodium carbonate was melted with PEG6000 (the amount of PEG6000 used was 5 wt% of sodium carbonate), then encapsulated and fed into an oven, dried at 40°C and then crushed to prepare a 80-mesh alkali source for use.

The raw material PEG-6000 was additionally weighed, placed in a superfine crushing vibration mill, superfinely crushed at below 40°C for 5 min and then taken out for use.

102 kg of the lyophilized powder of black Chinese wolfberry extract prepared above was weighed, firstly added together with 20 kg of citric acid to the superfine crushing vibration mill and superfinely mixed for 5 min at a temperature kept at 40°C; the superfinely mixed material was taken out, placed in a trough-type mixer, added with 12 kg of a water-soluble starch and mixed for 30 min; the resulting mixture was prepared into a soft material by adding 85% (V/V) ethanol (in an amount of 30% of the powder amount) as a wetting agent; a 15-mesh standard sieve was mounted on an oscillating granulator to prepare the soft material into granules; the granules was fed into a hot air circulating oven based on 1 kg/tray and dried at 40°C until the water content was about 3.0%; and the oven-dried granules was taken out and placed in a 15-mesh oscillating granulator for granulation. Subsequently, the resulting granules were mixed with 16 kg of the crushed alkali source, 46 kg of the remaining citric acid, 2 kg of sucralose and 2 kg of the superfinely crushed PEG-6000 in a three-dimensional mixer for 1 h; and the fully mixed granules were taken out, fed into a tablet press, and prepared into the black Chinese wolfberry effervescent tablet of this Example at an ambient humidity of ≤30%, a temperature of 20°C, a tablet press pressure of 9 kN and a rotational speed of 10 r/min.

### Example 3

Dried fruits of black Chinese wolfberry, citric acid, sodium carbonate, PEG6000, a water-soluble starch and sucralose were taken as raw materials for use.

A proper amount of harvested dried fruits of black Chinese wolfberry which had been washed clean and freed of impurities were weighed and added to an aqueous citric acid solution having a weight 3-5 times that of the dried fruits and a pH of about 3.5; the raw material was continuously stirred until the aqueous solution changed color, then cold-soaked for half an hour and then heated up to microboiling (about 90°C) for 1 h extraction; then a filtrate was collected through filtration; a filter residue was then added to an aqueous citric acid solution having a weight 3-5 times that of the filter residue and a pH of about 3.5; the raw material was continuously stirred and heated up to 90°C for 40 min extraction; then a filtrate was collected through filtration; and the two filtrates collected were combined and concentrated followed by lyophilization and then superfine crushing to above 300 meshes to prepare lyophilized powder of black Chinese wolfberry extract for use.

Sodium carbonate and PEG-6000 were weighed; and sodium carbonate was melted with PEG6000 (the amount of PEG6000 used was 5 wt% of sodium carbonate), then encapsulated and fed into an oven, dried at 40°C and then crushed to prepare a 80-mesh alkali source for use.

The raw material PEG-6000 was additionally weighed, placed in a superfine crushing vibration mill, superfinely crushed at below 40°C for 5 min and then taken out for use. 60 kg of the lyophilized powder of black Chinese wolfberry extract prepared above was weighed, firstly added together with 18 kg of citric acid to the superfine crushing vibration mill and superfinely mixed for 5 min at a temperature kept at 40°C; the superfinely mixed material was taken out, placed in a trough-type mixer, added with 4 kg of a water-soluble starch and mixed for 30 min; the resulting mixture was prepared into a soft material by adding 85% (V/V) ethanol (in an amount of 30% of the powder amount) as a wetting agent; a 15-mesh standard sieve was mounted on an oscillating granulator to prepare the soft material into granules; the granules was fed into a hot air circulating oven based on 1 kg/tray and dried at 40°C until the water content was about 3.0%; and the oven-dried granules was taken out and placed in a 15-mesh oscillating granulator for granulation.

Subsequently, the resulting granules were mixed with 9 kg of the crushed alkali source, 7 kg of the remaining citric acid, 1 kg of sucralose and 1 kg of the superfinely crushed PEG-6000 in a three-dimensional mixer for 1 h; and the fully mixed granules were taken out, fed into a tablet press, and prepared into the black Chinese wolfberry effervescent tablet of this Example at an ambient humidity of ≤30%, a temperature of 20°C, a tablet press pressure of 9 kN and a rotational speed of 10 r/min.

### Comparative Example 1

Dried fruits of black Chinese wolfberry, citric acid, sodium carbonate, PEG6000, a water-soluble starch and sucralose were taken as raw materials for use.

A proper amount of harvested dried fruits of black Chinese wolfberry which had been washed clean and freed of impurities were weighed and added to an aqueous citric acid solution having a weight 3-5 times that of the dried fruits and a pH of about 3.5; the raw material was continuously stirred until the aqueous solution changed color, then cold-soaked for half an hour and then heated up to microboiling (about 90°C) for 1 h extraction; then a filtrate was collected through filtration; a filter residue was then added to an aqueous citric acid solution having a weight 3-5 times that of the filter residue and a pH of about 3.5; the raw material was continuously stirred and heated up to 90°C for 40 min extraction; then a filtrate was collected through filtration; and the two filtrates collected were combined and concentrated followed by lyophilization and then superfine crushing to above 300 meshes to prepare lyophilized powder of black Chinese wolfberry extract for use.

Sodium carbonate and PEG-6000 were weighed; and sodium carbonate was melted with PEG6000 (the amount of PEG6000 used was 5 wt% of sodium carbonate), then encapsulated and fed into an oven, dried at 40°C and then crushed to prepare a 80-mesh alkali source for use.

The raw material PEG-6000 was additionally weighed, placed in a superfine crushing vibration mill, superfinely crushed at below 40°C for 5 min and then taken out for use. 51 kg of the lyophilized powder of black Chinese wolfberry extract prepared above was weighed, firstly added together with 33 kg of citric acid to the superfine crushing vibration mill and superfinely mixed for 5 min at a temperature kept at 40°C; the superfinely mixed material was taken out, placed in a trough-type mixer, added with 6 kg of a water-soluble starch and mixed for 30 min; the resulting mixture was prepared into a soft material by adding 85% (V/V) ethanol (in an amount of 30% of the powder amount) as a wetting agent; a 15-mesh standard sieve was mounted on an oscillating granulator to prepare the soft material into granules; the granules was fed into a hot air circulating oven based on 1 kg/tray and dried at 40°C until the water content was about 3.0%; and the oven-dried granules was taken out and placed in a 15-mesh oscillating granulator for granulation.

Subsequently, the resulting granules were mixed with 8 kg of the crushed alkali source, 1 kg of sucralose and 1 kg of the superfinely crushed PEG-6000 in a three-dimensional mixer for 1 h; and the fully mixed granules were taken out, fed into a tablet press, and prepared into the black Chinese wolfberry effervescent tablet at an ambient humidity of ≤30%, a temperature of 20°C, a tablet press pressure of 9 kN and a rotational speed of 10 r/min.

### Comparative Example 2

Dried fruits of black Chinese wolfberry, citric acid, sodium carbonate, PEG6000, a water-soluble starch and sucralose were taken as raw materials for use.

A proper amount of harvested dried fruits of black Chinese wolfberry which had been washed clean and freed of impurities were weighed and added to an aqueous citric acid solution having a weight 3-5 times that of the dried fruits and a pH of about 3.5; the raw material was continuously stirred until the aqueous solution changed color, then cold-soaked for half an hour and then heated up to microboiling (about 90°C) for 1 h extraction; then a filtrate was collected through filtration; a filter residue was then added to an aqueous citric acid solution having a weight 3-5 times that of the filter residue and a pH of about 3.5; the raw material was continuously stirred and heated up to 90°C for 40 min extraction; then a filtrate was collected through filtration; and the two filtrates collected were combined and concentrated followed by lyophilization and then superfine crushing to above 300 meshes to prepare lyophilized powder of black Chinese wolfberry extract for use.

Sodium carbonate and PEG-6000 were weighed; and sodium carbonate was melted with PEG6000 (the amount of PEG6000 used was 5 wt% of sodium carbonate), then encapsulated and fed into an oven, dried at 40°C and then crushed to prepare a 80-mesh alkali source for use.

The raw material PEG-6000 was additionally weighed, placed in a superfine crushing vibration mill, superfinely crushed at below 40°C for 5 min and then taken out for use. 60 kg of the lyophilized powder of black Chinese wolfberry extract prepared above was weighed, added to the superfine crushing vibration mill and superfinely crushed for 5 min at a temperature kept at 40°C; the superfinely crushed lyophilized powder was taken out, placed in a trough-type mixer, added with 4 kg of a water-soluble starch and mixed for 30 min; the resulting mixture was prepared into a soft material by adding 85% (V/V) ethanol (in an amount of 30% of the powder amount) as a wetting agent; a 15-mesh standard sieve was mounted on an oscillating granulator to prepare the soft material into granules; the granules was fed into a hot air circulating oven based on 1 kg/tray and dried at 40°C until the water content was about 3.0%; and the oven-dried granules was taken out and placed in a 15-mesh oscillating granulator for granulation.

Subsequently, the resulting granules were mixed with 9 kg of the crushed alkali source, 25 kg of citric acid, 1 kg of sucralose and 1 kg of the superfinely crushed PEG-6000 in a three-dimensional mixer for 1 h; and the fully mixed granules were taken out, fed into a tablet press, and prepared into the black Chinese wolfberry effervescent tablet at an ambient humidity of ≤30%, a temperature of 20°C, a tablet press pressure of 9 kN and a rotational speed of 10 r/min.

### Determination of anthocyanin

Reagent: 2% hydrochloric acid-methanol: hydrochloric acid (AR): methanol (AR)=2:98 (V/V) Instruments and apparatuses: an electronic balance, an ultrasonic cleaner, an ultraviolet spectrophotometer and a cuvette (1 cm)

Samples of the black Chinese wolfberry effervescent tablets of Examples 1 to 3 and Comparative Examples 1 to 2 were respectively taken, placed in a thermostat having a relative humidity of 30% RH and a temperature of 40°C for 30 days, then taken out and crushed; 20 mg of each of the samples of the black Chinese wolfberry effervescent tablets was then precisely weighed and added with 50 ml of 2% hydrochloric acid-methanol for 20 min ultrasonic dissolution; and the resulting respective solutions were respectively taken out, cooled, made up to 100 ml, evenly shaken and filtered for testing. Their absorbance A was determined at a wavelength of 530 nm by a 1 cm quartz cuvette, and a 2% hydrochloric acid-methanol solution was used a blank control. In this method, specific delphinidin in anthocyanin was used as a marker component for determination. The determination results are as shown in Table 1.

**Table 1: content of anthocyanin (%)**

| Example 1 | Example 2 | Example 3 | Comparative Example 1 | Comparative Example 2 |
|---|---|---|---|---|
| 1.53 | 1.55 | 1.62 | 0.92 | 0.65 |

### Color observation

One piece of each of the effervescent tablets (500 mg/piece) of Examples 1 to 3 was taken and placed in 100 mL of mineral water. It could be observed that the effervescent tablets disintegrated rapidly and the solutions had constantly changing color and finally became stable pink.

One piece of the effervescent tablet (500 mg/piece) of Comparative Example 1 was taken and placed in 100 mL of mineral water. It could be observed that the effervescent tablet constantly became dark and finally appeared dark red.

One piece of the effervescent tablet (500 mg/piece) of Comparative Example 2 was taken and placed in 100 mL of mineral water. It could be observed that the effervescent tablet constantly became dark and finally appeared dark purple.

In a granulation method, the raw materials defined in the invention are used for preparation, and lyophilized powder of black Chinese wolfberry is mixed with part of citric acid (1/4-3/4 of the total amount of citric acid) for superfine crushing, because anthocyanin contains acylated anthocyanin, and such acylated anthocyanin can protect the color stability of anthocyanin by its superfine crushing with citric acid. The addition of the remaining citric acid powder (the remaining 1/4 to 3/4) upon granulation can further enhance the protection for the stable acylated anthocyanin. After the effervescent tablet of the present application effervesces, its solution has constantly changing color, can present colorful bubbles and finally become stable pink. The black Chinese wolfberry effervescent tablet is interesting for such color change, has strong visual impact upon disintegration, can present gorgeous liquid color and has good taste with very attractive market prospects.

For the ordinary persons skilled in the art, particular examples are merely used to describe the invention in an exemplary manner. It is apparent that the specific implementation of the invention is not limited by the above manner, and various insubstantial improvements made based on the concepts and technical solutions of the method of the invention, or the direct application of the concepts and technical solution of the invention to other occasions without improvement should fall within the protection scope of the invention.

## Claims

1. A preparation method of a black Chinese wolfberry effervescent tablet prepared from lyophilized powder of black Chinese wolfberry extract, citric acid, an alkali source, a water-soluble starch, sucrose and an encapsulant as raw materials comprises the following steps:
(1) lyophilized powder of black Chinese wolfberry extract and part of citric acid are crushed and mixed;
(2) the crushed and mixed material is taken out, added with a water-soluble starch and mixed again, and the resulting mixture is prepared into a soft material by taking ethanol as a wetting agent, then prepared into granules and dried until the water content is controlled at 2.2-3.5%; and
(3) the granules prepared in step (2) are mixed with an alkali source, the remaining citric acid, sucrose and an encapsulant and then pressed into effervescent tablets.

2. The preparation method of a black Chinese wolfberry effervescent tablet according to claim 1, wherein the raw materials consist of 25-45% of citric acid, 5-10% of an alkali source, 3-7% of a water-soluble starch, 1-3% of an encapsulant, 0.5-5% of sucrose and 40-60% of lyophilized powder of black Chinese wolfberry extract based on the weight percentage.

3. The preparation method of a black Chinese wolfberry effervescent tablet according to claim 1, wherein the amount of citric acid used in step (1) is 1/4-3/4 of the total weight of citric acid.

4. The preparation method of a black Chinese wolfberry effervescent tablet according to claim 2, wherein the raw materials consist of 25-35% of citric acid, 8-10% of an alkali source, 3-7% of a water-soluble starch, 1-3% of an encapsulant, 0.5-5% of sucrose and 51-60% of lyophilized powder of black Chinese wolfberry extract.

5. The preparation method of a black Chinese wolfberry effervescent tablet according to claim 1, wherein the lyophilized powder of black Chinese wolfberry extract is prepared as follows: dried fruits of black Chinese wolfberry as a raw material are extracted with an aqueous citric acid solution having a pH value of about 3.5 and filtered to collect a filtrate, and the filtrate is concentrated followed by lyophilization.

6. The preparation method of a black Chinese wolfberry effervescent tablet according to claim 5, wherein the lyophilized powder of black Chinese wolfberry extract is prepared as follows: dried fruits of black Chinese wolfberry as a raw material are added to an aqueous citric acid solution having a weight 3-5 times that of the dried fruits of black Chinese wolfberry and a pH of about 3.5 while stirring, soaked at normal temperature for half an hour and then heated up to about 90°C for extraction, then a filtrate is collected through filtration, a filter residue is then added to an aqueous citric acid solution having a weight 3-5 times that of the filter residue and a pH of about 3.5, the raw material is continuously stirred and heated up to about 90°C for extraction, then a filtrate is collected through filtration, and the two filtrates collected are combined and concentrated followed by lyophilization and superfine crushing to above 300 meshes.

7. The preparation method of a black Chinese wolfberry effervescent tablet according to claim 1 or 2, wherein the alkali source is prepared as follows: sodium carbonate and/or sodium bicarbonate are/is melted with polyethylene glycol, then encapsulated and fed into an oven, dried at below 40°C and then crushed, wherein the amount of the polyethylene glycol used is 5-10 wt% of the sodium carbonate and/or sodium bicarbonate.

8. The preparation method of a black Chinese wolfberry effervescent tablet according to claim 1, wherein the encapsulant is superfinely crushed polyethylene glycol; and the superfinely crushed polyethylene glycol is prepared by superfine crushing of sheet-like polyethylene glycol at a temperature of below 40°C for more than 5 min using a superfine crushing vibration mill.

9. The preparation method of a black Chinese wolfberry effervescent tablet according to claim 1, wherein the sucrose is sucralose.

10. A black Chinese wolfberry effervescent tablet prepared by the preparation method according to any one of claims 1 to 9.
